# EUROPEAN PATENT SPECIFICATION

(11) **EP 2 979 685 B1**
(45) Date of publication and mention of the grant of the patent: **12.07.2017**
(21) Application number: 14178979.2
(22) Date of filing: 29.07.2014
(51) Int. Cl.: A61K 8/73, A61K 8/26, A61Q 19/00

(54) **Film forming complex, cosmetic composition comprising it and their use as peel-off film mask**
Filmbildungskomplex, kosmetische Zusammensetzung damit und Verwendung davon als abziehbare Filmmaske
Complexe de formation de film, composition cosmétique le comprenant et son utilisation en tant que masque de film retirable

(43) Date of publication of application: 03.02.2016
(73) Proprietor: COTY GERMANY GMBH, 55116 Mainz (DE)
(72) Inventor: MOYON, Roselyne, 06110 Le Cannet (FR); PISSAVINI, Marc, 06790 Aspremont (FR); DOUCET, Oliver, 06190 Roquebrune Cap Martin (FR)
(74) Representative: Gulde & Partner

(56) References cited:
- EP-A1- 1 887 030
- WO-A2-03/028635
- US-A1- 2005 058 613
- DATABASE GNPD [Online] MINTEL; November 2012 (2012-11), "Night gel", XP002734177, Database accession no. 1900222
- DATABASE GNPD [Online] MINTEL; May 2013 (2013-05), "Stage release eye moisturiser", XP002734178, Database accession no. 2044688
- DATABASE GNPD [Online] MINTEL; September 2013 (2013-09), "Tightening face cream", XP002734179, Database accession no. 2155983
- DATABASE GNPD [Online] MINTEL; March 2010 (2010-03), "Clarifying peel-off mask", XP002734180, Database accession no. 1288881
- DATABASE GNPD [Online] MINTEL; March 2014 (2014-03), "Vital energy infusion mask", XP002734181, Database accession no. 2511481

## Description

The present invention relates to a film forming complex and a cosmetic composition comprising this complex, both with excellent peel-off properties. The film forming complex comprises two natural film formers which are Hydrolyzed Corn Starch (trade name Asensa NFF11E) and Pullulan (trade name Pullulan). Both, the complex of the invention and the cosmetic composition comprising the complex do not contain polyvinyl alcohol, alginates, alginic acid, polyurethane and ethanol. If the film forming complex or the cosmetic composition of the invention is applied to the skin a thin film mask with excellent coverage is formed within a drying time of less than 10 to 15 minutes, preferably 11 minutes. Following treatment, the mask will peel off easily in a single piece without pain. It does not cause redness and leaves a comfortable, smooth and pleasant feel on the skin.

Peel-off facial masks are well known in the prior art. They are used to clean the skin, to tense it, to moisturize it, to remove dead cells, residues and other materials deposited on the *stratum corneum.* As film forming agent polyvinyl alcohol is widely used. For instance EP 1 186 291 B1 discloses a cosmetic composition with 11% polyvinyl alcohol, 12,5% ethanol and 4% PVP/VA-Copolymer which is useful as cleansing mask. A commercially available product with polyvinyl alcohol and ethanol is the clarifying peel-off mask of Club des Createurs de Beaute/ Cosmense (France).

Alginates are also known to be used in peel-off masks. An especially preferred product is Isagel™FM alginate (INCI name: diatomeous earth (and) alginate (and) calcium sulfate (and) tetrasodium pyrophosphate) of FMC BioPolymer (U.S.A.). A peel-off type cosmetic with sodium alginate is described in JP 04021620 A.

A synthetic film forming polymer which is described to be useful for any type of leave-on cosmetic formulations is Baycusan C 1003 (INCI name: Polyurethane - 32). It is described to perform exceptionally well as a film former in face mask applications.

It was the object of the present invention to provide a pleasant and comfortable peel-off cosmetic mask for skin care which is at least as effective as the film forming systems known in the prior art, but which does not contain the conventional film forming raw materials like polyvinyl alcohol, alginates, alginic acid and polyurethane, but uses natural film formers. Additionally, the composition of the invention should not contain ethanol which is known to cause irritations on sensitive skin.

The inventors of the present invention have found that a film forming complex consisting of
0,1 to 20 wt% Hydrolyzed Corn Starch,
0,1 to 20 wt% Pullulan,
0,1 to 3 wt% of a rheology modulating polymer,
0,1 to 20 wt% of a plasticizer,
0,1 to 30 wt% kaolin,
water and cosmetically acceptable auxiliaries provides a basic formulation which fulfils all main characteristics of a peel-off film mask. The given percentages relate to the total weight of the film forming complex. The complex does not contain polyvinyl alcohol, polyurethane, alginates, alginic acid and ethanol. The Hydrolyzed Corn Starch is of "green" origin (99% bio-based from starch biopolymer). The Pullulan has also the advantage to be a 100% vegetable origin natural polysaccharide. It is produced by fermentation of plant pulps, and it is totally biodegradable.

Hydrolyzed Corn Starch (trade name: Asensa®NFF 11E, Honeywell International, Inc.) can be formulated in water based systems without the use of solvents and it has occlusive properties. In a preferred embodiment of the invention the Hydrolyzed Corn Starch is contained in the film forming complex in the range of 2 to 18 wt%.

Pullulan is known to be a film former with excellent adhesive properties when dried. It is preferably contained in the film forming complex from 2 to 18wt%.

According to the invention a rheology modulating polymer was added to the film forming complex to adjust easily the texture of the final product and to ensure the homogeneity of the film on the skin. For that purpose a huge number of thickeners or viscosity regulating substances come into consideration. For instance naturally derived polymers like e.g. long-chained and branched carbohydrates as e.g. guar gum, locust bean gum, xanthan gum, Konjac mannan or gellan gum, can be used. In a preferred embodiment of the invention the rheology modulating polymer is a synthetic polymer, especially an acrylic polymer or acrylic copolymer. It is particularly preferred that the acrylic polymer is carbomer. As acrylic copolymer a copolymer of sodium hydroxyethyl acrylate and acryloyldimethyl taurate monomers is preferred, especially preferred is Hydroxyethylacrylate & Sodium Acryloyldimethyltaurate Copolymer & Squalane & Polysorbate 60 (INCI; trade name: Simulgel NS). It turned out that Simulgel NS also decreased the drying time of the film on the skin and the apparition of redness on the skin after film removal.

In a preferred embodiment of the invention the rheology modulating polymer is contained in the film forming complex in the range of 1.0 to 3.0 wt%.

According to the invention the film forming complex also comprises a plasticizer to ensure good removal abilities of the film. Suitable plasticizers for cosmetic films are for instance C₁-C₂₀-esters of malic acid, e.g. dibutyl or dioctyl maleate, trioctyldodecyl citrate or glycerine triacetate. In a preferred embodiment of the invention a polyethylenglycol coconut fatty acid ester, especially polyoxyethylene glyceryl monococoate (INCI: PEG-7 Glyceryl Cocoate; trade name: Cetiol®HE) is used. This hydrophilic oil has refatting properties and does not only act as plasticizer, but improves on top of that the after skin feeling.

In a preferred embodiment of the invention the plasticizer is contained in the film forming complex in the range of 2 to 16 wt%.

Kaolin (Hydrated Aluminium Silicate, Brenntag Specialties, Inc.; synonym: kaolin clay; trade name: Kaolin Colloidal USP BC 2457) is preferably contained in the film forming complex in the range of 1.0 to 20 wt%.

In an especially preferred embodiment of the invention the film forming complex may contain Carrageenan which surprisingly further improves the film adhesion to the skin. Carrageenan is a gel-forming polysaccharide found in the "red algae" seaweed known as "Irish moss". It is commercially available as e.g. GELCARIN^{®} (Trade name) from FMC BioPolymer. It may be contained in the film forming complex from 0.1 to 5 wt% relating to the total weight of the complex.

The cosmetically acceptable auxiliaries which are contained in the film forming complex of the invention comprise at least a base, preferably triethanolamin, glycerol and a preservative.

The film forming complex of the invention can be prepared according to methods which are basically known to skilled experts. Water is heated, the base, preferably triethanolamin, and the Hydrolyzed Corn Starch are added under stirring until a clear amber solution appears. Then the Pullulan powder is dispersed in the obtained solution under stirring until a syrupy clear fluid is obtained. Glycerol, plasticizer and preservative are added under stirring, and kaolin powder is dispersed in the solution under stirring until the product becomes uniformly opaque. Finally the rheology modulating polymer is added under stirring until the gel has a viscosity from 40000 to 100000 mPa*s.

The combination of the two selected film formers, the rheology modulating polymer, the plasticizer and the kaolin permits to get a film with all main characteristics required by a peel-off film mask:
- adapted rheological behaviour to get a thin and regular film on the skin,
- a quick time to dry (less than 10-15min) to transform the product in a gel film,
- a good removal properties (supple and elastic film), not painful,
- does not cause redness and leaves a comfortable after feel on the skin.,
- permits the introduction of various active ingredients.

It is one advantage of the described film forming complex that it can be modified by including desired active skin care ingredients and auxiliaries such as for instance fragrances, colorants and pigments. Therefore, a cosmetic composition which is suitable as peel-off film mask comprising 0.10 to 99.0 wt% of the film forming complex and comprising cosmetically active ingredients and cosmetically acceptable auxiliaries up to 100 wt% represents a further object of the present invention, wherein the given percentages relate to the total weight of the cosmetic composition.

In one preferred embodiment of the invention the cosmetically active ingredients of the cosmetic composition are ingredients for skin care, preferably one or more ingredients selected from the group consisting of ingredients for purifying the skin, brightening the skin, smoothing the skin, matting the skin, moisturizing the skin, firming the skin, whitening the skin or delivering a natural and radiant tan. Such active ingredients are well known to the skilled expert.

Ingredients for purifying the skin which can be used according to the invention are for instance zinc oxide, zinc salts and derivates, algae extracts (e.g. Florogine from Biotechmarine) or botanical extracts known for this purpose.

Active ingredients for matting the skin which may be added according to the invention are for instance powders such as Greensil from Greentech or PTFE or botanical extracts as e.g. Witch hazel.

Active ingredients for brightening and whitening the skin which can be used according to the invention are for instance vitamin C glucoside, algae extracts (e.g. whitonyl) or botanical extracts as e.g. licorice extract.

Active ingredients for smoothing and moisturizing the skin which can be used according to the invention are for instance known botanical and marine extracts. An ingredient for moisturizing is for instance Lipidure PMB from NOF Corp..

For firming the skin peptides such as e.g. Calmosensine or Matrixyl from Sederma, retinoides, vitamin C derivates or known botanical extracts may be preferably used as active ingredients according to the invention.

As tanning ingredients for instance dihydroxyacetone, Heliotan complex from Lancaster, Biotanning from Silab or botanical extracts such as e.g. Urucum extract or green tea extract may be added according to the present invention.

The cosmetic composition of the invention is prepared by first preparing the film forming complex as described above and adding then the desired active ingredients at the end stage. If the ingredients are solid they can also be added during the heating stage.

The percentages of the actives have to be adapted in accordance with the intended application what is a usual procedure for the skilled expert.

A further object of the present invention is the cosmetic use of the film forming complex or the cosmetic composition of the invention as peel off skin mask, preferably as peel-off facial and/or body mask. According to the invention they are used for skin care, preferably for purifying the skin, brightening the skin, smoothing the skin, matting the skin, moisturizing the skin, firming the skin, whitening the skin or delivering a natural and radiant tan, preferably for purifying the skin.

The film forming complex for use in skin care and the cosmetic composition for use in skin care as well as a method for skin care by applying the complex or the cosmetic composition to skin areas to be treated are further objects of the present invention.

The following examples are offered to illustrate the film forming complex of the present invention. They are not intended to be limiting in any respect.

### Example 1:

| Film forming complexes comprising | (wt%) |
|---|---|
| Asensa NFF11E | 8 to 12 (8) |
| Pullulan | 8 to 12 (8) |
| Simulgel NS | 1.5 to 2.5 (1.5) |
| Cetiol HE | 5 to 11 (5) |
| Kaolin | 6 to 10 (6) |

were prepared.

The preparation was according to the below described process:
Distilled water is heated up to 55-65°C, triethanolamin, is added and the Hydrolyzed Corn Starch powder is dispersed therein under stirring, preferably with a propeller speed of 1100-1300 m⁻¹, until a clear amber solution appears, preferably during about 10 minutes. The heating is stopped and the Pullulan powder is dispersed in the obtained solution under stirring, with preferably the same propeller speed as above, until a syrupy clear fluid is obtained, preferably during about 10 minutes. Then, at 40-35°C glycerol, plasticizer and preservative are added under stirring, preferably with a propeller speed of 850-950 m⁻¹, preferably during about 2 minutes. Then kaolin powder is dispersed in the solution under stirring, preferably with a propeller speed of 760-1000 m⁻¹, until the product becomes uniformly opaque, preferably during about 5 minutes. Finally the rheology modulating polymer is added under stirring, preferably with a propeller speed of 650-750 m⁻¹, until the gel has a viscosity from 40000 to 100000 mPa*s, preferably during about 10 minutes.

### Example 2:

### Evaluation of the film forming complex of Example 1 versus benchmarks

The film forming properties of the complex of Example 1 were compared with three benchmarks:
- Benchmark 1 -: Formulation with Alginate (Isagel FM) as film former
- Benchmark 2 -: Formulation with Polyurethane-32 (Baycusan C 1003) as film former
- Benchmark 3 -: Formulation with PVA as film former (Clarifying Peel off mask of Cosmense "White Defense") which is available on the cosmetic market

### Preparation of the Alginate formulation of benchmark 1:

27wt% of Isagel FM, water qs, and preservative were mixed to give the formulation of benchmark 1. Water (with preservative and optionally with actives, if they are liquid) is added to Isagel (optionally with actives, if they are in powder form), and then it is vigorously mixed during 1 min.

### Preparation of the Polyurethane formulation of benchmark 2:

46 wt% polyurethane, water, preservative, 4% glycerol, 2% Cetiol HE as plasticizer and 1.2% xanthan gum were formulated to give the formulation of benchmark 2. All ingredients are mixed until a completely uniform and homogeneous solution is obtained.

The comparison was performed as follows:
The same quantity of each formulation (259mg) was applied with a spatula onto the inside arm (4*5cm) of 3 women. The quantity was chosen to get the most regular film on delimited areas.

The investigated film and skin properties were the following:
drying time of the film: quick<5min, correct=8-10min, too long ≥15min
easiness of removal of the film from the skin: very easy, easy, difficult, painful condition of the removed film: supple, elastic, pieces, uniform
residues on the skin: no, little, a lot
immediate after skin aspect (condition): red, matte, looks thinner
skin afterfeel: uncomfortable, tighten, comfortable

### Results:

It revealed that the film forming complex of Example 1 was superior to the Alginate and Polyurethane formulations (benchmarks 1 and 2). It showed equivalent behaviour to the market product (benchmark 3) with regard to all characteristics.

## Claims

1. A film forming complex consisting of
0,1 to 20 wt% Hydrolyzed Corn Starch,
0,1 to 20 wt% Pullulan,
0,1 to 3 wt% of a rheology modulating polymer,
0,1 to 20 wt% of a plasticizer,
0,1 to 30 wt% kaolin,
water and cosmetically acceptable auxiliaries, whereby all given percentages relate to the total weight of the film forming complex and
wherein the film forming complex does not contain polyvinyl alcohol, polyurethane, alginates, alginic acid and ethanol.

2. The film forming complex according to claim 1, **characterized in that** Hydrolyzed Corn Starch is contained in the range of 2.0 to 18 wt%.

3. The film forming complex according to claim 1 or 2, **characterized in that** Pullulan is contained in the range of 2.0 to 18 wt%.

4. The film forming complex according to any one of claims 1 to 3, **characterized in that** the rheology modulating polymer is an acrylic polymer, preferably carbomer, or an acrylic copolymer, preferably a copolymer of sodium hydroxyethyl acrylate and acryloyldimethyl taurate monomers, more preferred Hydroxyethylacrylate & Sodium Acryloyldimethyltaurate Copolymer & Squalane & Polysorbate 60.

5. The film forming complex according to any one of claims 1 to 4, **characterized in that** the rheology modulating polymer is contained in the range of 1.0 to 3.0 wt%.

6. The film forming complex according to any one of claims 1 to 5, **characterized in that** the plasticizer is contained in the range of 2 to 16 wt%.

7. The film forming complex according to any one of claims 1 to 6, **characterized in that** the plasticizer is a polyethylenglycol coconut fatty acid ester, preferably polyoxyethylene glyceryl monococoate.

8. The film forming complex according to any one of claims 1 to 7, **characterized in that** kaolin is contained in the range of 1 to 20 wt%.

9. The film forming complex according to any one of claims 1 to 8, **characterized in that** Carrageenen is contained in the complex, preferably from 0.1 to 5 wt% related to the total weight of the complex.

10. The film forming complex according to any one of claims 1 to 9, **characterized in that** the cosmetically acceptable auxiliaries comprise triethanolamine, glycerol and preservative.

11. A cosmetic composition with peel-off properties comprising 0.10 to 99.0 wt% of a film forming complex according to any one of claims 1 to 10 and further comprising cosmetically active ingredients and cosmetically acceptable auxiliaries up to 100 wt%, whereby the given percentages relate to the total weight of the composition, and wherein the composition does not contain polyvinyl alcohol, polyurethane, alginates, alginic acid and ethanol.

12. The cosmetic composition according to claim 11, **characterized in that** the cosmetically active ingredients are one or more ingredients for skin care, preferably selected from the group consisting of ingredients for purifying the skin, brightening the skin, smoothing the skin, matting the skin, moisturizing the skin, firming the skin, whitening the skin or delivering a natural and radiant tan.

13. Cosmetic use of a film forming complex according to any one of claims 1 to 10 or of a composition according to claim 11 or 12 as peel off skin mask, preferably as peel-off facial and/or body mask.

14. Cosmetic use according to claim 13, **characterized in that** the peel off skin mask is used for skin care, preferably for purifying the skin, brightening the skin, smoothing the skin, matting the skin, moisturizing the skin, firming the skin, whitening the skin or delivering a natural and radiant tan, preferably for purifying the skin.

15. A method for skin care **characterized in that** a film forming complex according to any one of claims 1 to 10 or a cosmetic composition according to claim 11 or 12 is applied to the skin areas to be treated.

## Patentansprüche

1. Filmbildungskomplex, bestehend aus:
0,1 bis 20 Gew.-% Hydrolyzed Corn Starch,
0,1 bis 20 Gew.-% Pullulan,
0,1 bis 3 Gew.-% eines das Fließverhalten modulierenden Polymers,
0,1 bis 20 Gew.-% eines Weichmachers,
0,1 bis 30 Gew.-% Kaolin,
Wasser und kosmetisch annehmbaren Hilfsstoffen, wobei sich alle angegebenen Prozentsätze auf das Gesamtgewicht des Filmbildungskomplexes beziehen und wobei der Filmbildungskomplex keinen Polyvinylalkohol, kein Polyurethan, keine Alginate, keine Alginsäure und kein Ethanol enthält.

2. Filmbildungskomplex nach Anspruch 1, **dadurch gekennzeichnet, dass** Hydrolyzed Corn Starch im Bereich von 2,0 bis 18 Gew.-% enthalten ist.

3. Filmbildungskomplex nach Anspruch 1 oder 2, **dadurch gekennzeichnet, dass** Pullulan im Bereich von 2,0 bis 18 Gew.-% enthalten ist.

4. Filmbildungskomplex nach einem der Ansprüche 1 bis 3, **dadurch gekennzeichnet, dass** das das Fließverhalten modulierende Polymer ein Acrylpolymer, vorzugsweise Carbomer, oder ein Acrylcopolymer, vorzugsweise ein Copolymer aus Natriumhydroxyethylacrylat- und Acryloyldimethyltaurat-Monomeren, besonders bevorzugt Hydroxyethylacrylate & Sodium Acryloyldimethyltaurate Copolymer & Squalane & Polysorbate 60, ist.

5. Filmbildungskomplex nach einem der Ansprüche 1 bis 4, **dadurch gekennzeichnet, dass** das das Fließverhalten modulierende Polymer im Bereich von 1,0 bis 3,0 Gew.-% enthalten ist.

6. Filmbildungskomplex nach einem der Ansprüche 1 bis 5, **dadurch gekennzeichnet, dass** der Weichmacher im Bereich von 2 bis 16 Gew.-% enthalten ist.

7. Filmbildungskomplex nach einem der Ansprüche 1 bis 6, **dadurch gekennzeichnet, dass** der Weichmacher ein Polyethylenglykol-Kokosnussfettsäureester, vorzugsweise Polyoxyethylenglycerylmonococoat, ist.

8. Filmbildungskomplex nach einem der Ansprüche 1 bis 7, **dadurch gekennzeichnet, dass** Kaolin im Bereich von 1 bis 20 Gew.-% enthalten ist.

9. Filmbildungskomplex nach einem der Ansprüche 1 bis 8, **dadurch gekennzeichnet, dass** Carrageenan) in dem Komplex enthalten ist, vorzugsweise von 0,1 bis 5 Gew.-% bezogen auf das Gesamtgewicht des Komplexes.

10. Filmbildungskomplex nach einem der Ansprüche 1 bis 9, **dadurch gekennzeichnet, dass** die kosmetisch annehmbaren Hilfsstoffe Triethanolamin, Glycerin und ein Konservierungsmittel umfassen.

11. Kosmetische Zusammensetzung mit Abziehbarkeitseigenschaften, die 0,10 bis 99,0 Gew.-% eines Filmbildungskomplexes nach einem der Ansprüche 1 bis 10 umfasst und ferner kosmetisch wirksame Inhaltsstoffe und kosmetisch annehmbare Hilfsstoffe bis zu 100 Gew.-% umfasst, wobei sich die angegebenen Prozentsätze auf das Gesamtgewicht der Zusammensetzung beziehen und wobei die Zusammensetzung keinen Polyvinylalkohol, kein Polyurethan, keine Alginate, keine Alginsäure und kein Ethanol enthält.

12. Kosmetische Zusammensetzung nach Anspruch 11, **dadurch gekennzeichnet, dass** die kosmetisch wirksamen Inhaltsstoffe ein oder mehrere Inhaltsstoffe für die Hautpflege sind, vorzugsweise ausgewählt aus der Gruppe bestehend aus Inhaltsstoffen zum Reinigen der Haut, Aufhellen der Haut, Glätten der Haut, Mattieren der Haut, Befeuchten der Haut, Festigen der Haut, Bleichen der Haut oder Verleihen einer natürlichen und schönen Bräune.

13. Kosmetische Verwendung eines Filmbildungskomplexes nach einem der Ansprüche 1 bis 10 oder einer Zusammensetzung nach Anspruch 11 oder 12 als abziehbare Hautmaske, vorzugsweise als abziehbare Gesichts- und/oder Körpermaske.

14. Kosmetische Verwendung nach Anspruch 13, **dadurch gekennzeichnet, dass** die abziehbare Hautmaske für die Hautpflege verwendet wird, vorzugsweise zum Reinigen der Haut, Aufhellen der Haut, Glätten der Haut, Mattieren der Haut, Befeuchten der Haut, Festigen der Haut, Bleichen der Haut oder Verleihen einer natürlichen und schönen Bräune, vorzugsweise zum Reinigen der Haut.

15. Verfahren zur Hautpflege, **dadurch gekennzeichnet, dass** ein Filmbildungskomplex nach einem der Ansprüche 1 bis 10 oder eine kosmetische Zusammensetzung nach Anspruch 11 oder 12 auf die zu behandelnden Hautbereiche aufgetragen wird.

## Revendications

1. Complexe de formation de film, composé de
0,1 à 20 % en poids de Hydrolyzed Corn Starch,
0,1 à 20 % en poids de Pullulan,
0,1 à 3 % en poids d'un polymère modulateur de rhéologie,
0,1 à 20 % en poids d'un plastifiant,
0,1 à 30 % en poids de kaolin,
d'eau et d'auxiliaires cosmétiquement acceptables, tous les pourcentages indiqués étant relatifs au poids total du complexe de formation de film, et ledit complexe de formation de film ne contenant pas d'alcool polyvinylique, de polyuréthane, d'alginates, d'acide alginique, ni d'éthanol.

2. Complexe de formation de film selon la revendication 1, **caractérisé en ce que** la teneur de Hydrolyzed Corn Starch est comprise entre 2,0 et 18 % en poids.

3. Complexe de formation de film selon la revendication 1 ou la revendication 2, **caractérisé en ce que** la teneur de Pullulan est comprise entre 2,0 et 18 % en poids.

4. Complexe de formation de film selon l'une quelconque des revendications 1 à 3, **caractérisé en ce que** le polymère modulateur de rhéologie est un polymère acrylique, préférentiellement un carbomer, ou un copolymère acrylique, préférentiellement un copolymère d'acrylate d'hydroxyéthyle sodique et de monomères acryloyldiméthyltaurate, préférentiellement Hydroxyethylacrylate & Sodium Acryloyldimethyltaurate Copolymer & Squalane & Polysorbate 60.

5. Complexe de formation de film selon l'une quelconque des revendications 1 à 4, **caractérisé en ce que** la teneur de polymère modulateur de rhéologie est comprise entre 1,0 et 3,0 % en poids.

6. Complexe de formation de film selon l'une quelconque des revendications 1 à 5, **caractérisé en ce que** la teneur du plastifiant est comprise entre 2 et 16 % en poids.

7. Complexe de formation de film selon l'une quelconque des revendications 1 à 6, **caractérisé en ce que** le plastifiant est un ester d'acide gras polyéthylène-glycol de noix de coco, préférentiellement monococoate glycéryle de polyoxyéthylène.

8. Complexe de formation de film selon l'une quelconque des revendications 1 à 7, **caractérisé en ce que** la teneur de kaolin est comprise entre 1 et 20 % en poids.

9. Complexe de formation de film selon l'une quelconque des revendications 1 à 8, **caractérisé en ce que** du Carrageenan est contenu dans le complexe, préférentiellement entre 0,1 et 5 % en poids par rapport au poids total du complexe.

10. Complexe de formation de film selon l'une quelconque des revendications 1 à 9, **caractérisé en ce que** les auxiliaires cosmétiquement acceptables comprennent de la triéthanolamine, du glycérol et un conservateur.

11. Composition cosmétique présentant des propriétés de gommage, comprenant entre 0,10 et 99,0 % en poids du complexe de formation de film selon l'une des revendications 1 à 10 et comprenant en outre des ingrédients cosmétiquement actifs et des auxiliaires cosmétiquement acceptables jusqu'à 100 % en poids, les pourcentages indiqués étant relatifs au poids total de la composition, et ladite composition ne contenant pas d'alcool polyvinylique, de polyuréthane, d'alginates, d'acide alginique, ni d'éthanol.

12. Composition cosmétique selon la revendication 11, **caractérisé en ce que** les ingrédients cosmétiquement actifs sont un ou plusieurs ingrédients de soin pour la peau, sélectionnés préférentiellement dans le groupe composé d'ingrédients pour purifier la peau, éclaircir la peau, lisser la peau, matifier la peau, nourrir la peau, affermir la peau, blanchir la peau ou favoriser un bronzage naturel et éclatant.

13. Utilisation cosmétique d'un complexe de formation de film selon l'une des revendications 1 à 10 ou d'une composition selon la revendication 11 ou la revendication 12, en tant que masque de gommage cutané, préférentiellement en tant que masque de gommage pour le visage et/ou le corps.

14. Utilisation cosmétique selon la revendication 13, **caractérisé en ce que** le masque de gommage pour le visage est utilisé pour le soin de la peau, préférentiellement pour purifier la peau, éclaircir la peau, lisser la peau, matifier la peau, nourrir la peau, affermir la peau, blanchir la peau ou favoriser un bronzage naturel et éclatant, préférentiellement pour purifier la peau.

15. Procédé de soin pour la peau, **caractérisé en ce qu'**un complexe de formation de film selon l'une quelconque des revendications 1 à 10 ou une composition cosmétique selon la revendication 11 ou la revendication 12 sont appliqués sur des surfaces cutanées à traiter.
